# FASCICULE DE BREVET EUROPEEN

(11) **EP 1 769 798 B1**
(45) Date de publication et mention de la délivrance du brevet: **13.08.2008**
(21) Numéro de dépôt: 06291421.3
(22) Date de dépôt: 08.09.2006
(51) Int. Cl.: A61K 31/5375, A61K 31/165, A61P 25/24

(54) **Nouvelle association entre l'agomélatine et un inhibiteur de la recapture de la noradrénaline et les compositions pharmaceutiques qui la contiennent**
Neue Zusammenstellung von Agomelatin und einem Noradrenalin-Wideraufnahme-Hemmer und pharmazeutische Zusammensetzungen, die sie enthalten
New association of agomelatine and a noradrenalin reuptake inhibitor, and pharmaceutical compositions containing them

(30) Priorité: 09.09.2005 FR 0509206
(43) Date de publication de la demande: 04.04.2007
(73) Titulaire: Les Laboratoires Servier, 92415 Courbevoie Cedex (FR)
(72) Inventeur: Mocaer, Elisabeth, 92200 Neuilly sur Seine (FR)

(56) Documents cités:
- WO-A-20/05002562
- CHILMAN-BLAIR K ET AL: "AGOMELATINE. ANTIDEPRESSANT TREATMENT OF BIPOLAR DISORDER MELATONIN AGONUST/5-HT20 ANTAGONIST" DRUGS OF THE FUTURE, BARCELONA, ES, vol. 28, no. 1, janvier 2003 (2003-01), pages 7-13, XP009036058 ISSN: 0377-8282
- VERSIANI M: "THE SELECTIVE NORADRENALINE RE-UPTAKE INHIBITOR REBOXETINE HAS AN EARLY ONSET OF ANTIDEPRESSANT ACTION" INTERNATIONAL JOURNAL OF PSYCHIATRY IN CLINICAL PRACTICE, MARTIN DUNITZ, LONDON, GB, vol. 4, no. 4, 2000, pages 293-297, XP008039367 ISSN: 1365-1501
- FLEISHAKER J C: "CLINICAL PHARMACOKINETICS OF REBOXETINE, A SELECTIVE NOREPINEPHRINE REUPTAKE INHIBITOR FOR THE TREATMENT OF PATIENTS WITH DEPRESSION" CLINICAL PHARMACOKINETICS, LEA & FEBIGER, PHILADELPHIA, PA, US, vol. 39, no. 6, 2000, pages 413-427, XP000991558

## Description

La présente invention concerne une nouvelle association entre l'agomélatine ou *N*-[2-(7-méthoxy-1-naphtyl)éthyl]acétamide de formule (I) : 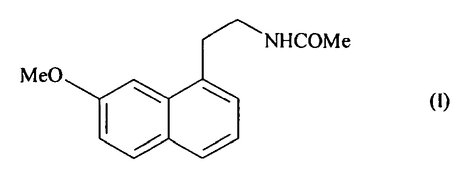 ou un de ses hydrates, formes cristallines et sels d'addition à un acide ou à une base pharmaceutiquement acceptable et un agent inhibiteur de la recapture de la noradrénaline ou tout agent capable d'augmenter la concentration en noradrénaline au niveau extracellulaire pour l'obtention de compositions pharmaceutiques utiles dans le traitement de la dépression et plus particulièrement dans le traitement des dépressions résistantes.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

La demanderesse a présentement découvert que l'agomélatine ou *N*-[2-(7-méthoxy-1-naphtyl)éthyl]acétamide ou un de ses hydrates, formes cristallines et sels d'addition à un acide ou à une base pharmaceutiquement acceptable, utilisée en association avec un agent inhibiteur de la recapture de la noradrénaline, possédait des propriétés intéressantes permettant de l'utiliser dans le traitement du trouble dépressif majeur, des dépressions saisonnières et plus particulièrement dans le traitement des dépressions résistantes, ainsi que dans le traitement de la comorbidité psychiatrique associée au trouble de l'humeur : anxiété, panique, stress, troubles du sommeil...

Les désordres du système nerveux central comme la dépression et l'anxiété affectent une population nombreuse et de tous âges. Bien qu'il existe un grand nombre de molécules efficaces dans ce domaine, aucune ne permet de guérir pleinement ces différents états pathologiques, et nombre d'entre elles présentent d'importants effets secondaires. Ainsi, l'élaboration de nouveaux traitements alternatifs est toujours d'actualité et reste une nécessité. Ceci est particulièrement vrai dans le cas de patients totalement réfractaires à tout traitement.
Une stratégie classique dite « d'association » consiste à combiner plusieurs traitements ayant des mécanismes d'action différents afin d'obtenir un effet positif. En règle générale, l'effet bénéfique observé est patients-dépendant et provient de la réaction à l'un ou l'autre des composés de l'association.

Les associations les plus classiques décrites dans la littérature concernent des associations avec des stabilisateurs d'humeur comme le lithium, avec d'autres antidépresseurs soit de profil clinique différent soit de profil neurochimique différent.

La demanderesse a présentement découvert que, de façon surprenante, les effets de l'agomélatine ou un de ses hydrates, formes cristallines et sels d'addition à un acide ou à une base pharmaceutiquement acceptable sont potentialisés par les agents inhibiteurs de la recapture de la noradrénaline, ou les agents capables d'augmenter les concentrations en noradrénaline au niveau extracellulaire. Ces agents présentent la particularité de potentialiser les effets de l'agomélatine.

Cet effet, non prévisible, permet d'envisager l'utilisation de l'association dans le traitement du trouble dépressif majeur, des dépressions saisonnières et plus particulièrement dans le traitement des dépressions résistantes, ainsi que de la comorbidité psychiatrique associée au trouble de l'humeur : anxiété, panique, stress, troubles du sommeil. Encore plus particulièrement, cette potentialisation des effets permettra d'utiliser l'association selon l'invention dans le traitement de patients réfractaires à tout traitement.

Parmi les agents inhibiteurs de la recapture de la noradrénaline selon l'invention, on peut citer plus particulièrement à titre non limitatif la réboxétine et la desipramine.

L'agent inhibiteur de la recapture de la noradrénaline préféré selon l'invention est la réboxétine.

L'invention concerne donc l'utilisation de l'association entre l'agomélatine ou un de ses hydrates, formes cristallines et sels d'addition à un acide ou à une base pharmaceutiquement acceptable et un composé inhibiteur de la recapture de la noradrénaline ou capable d'augmenter la concentration en noradrénaline au niveau extracellulaire, pour l'obtention de compositions pharmaceutiques destinées au traitement du trouble dépressif majeur, des dépressions saisonnières et plus particulièrement des dépressions résistantes, ainsi que dans le traitement de la comorbidité psychiatrique associée au trouble de l'humeur : anxiété, panique, stress, troubles du sommeil.

L'invention concerne également les compositions pharmaceutiques contenant l'association entre l'agomélatine ou un de ses hydrates, formes cristallines et sels d'addition à un acide ou à une base pharmaceutiquement acceptable et un composé inhibiteur de la recapture de la noradrénaline ou capable d'augmenter la concentration en noradrénaline au niveau extracellulaire, en combinaison avec un ou plusieurs excipients pharmaceutiquement acceptable.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

Outre l'agomélatine et le composé inhibiteur de la recapture de la noradrénaline, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

### A titre d'exemple et de manière non limitative, on peut citer :

◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures et plus préférentiellement 25 mg par jour. La dose de l'agent inhibiteur de la recapture de la noradrénaline sera moindre que celle utilisée lorsqu'il est administré seul.

### Composition pharmaceutique :

**Formule de préparation pour 1000 comprimés dosés à 25 mg :**

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude clinique :

Une étude clinique est réalisée chez des patients présentant un trouble dépressif majeur traités soit par agomélatine et placébo soit par agomélatine et réboxétine sur une durée de 6 semaines. Le système diagnostique utilisé est celui du DSM-IV ; le critère principal d'efficacité est le score total de l'échelle de dépression de Hamilton. Un recueil des événements indésirables est réalisé. L'étude montre une activité supérieure de l'association agomélatine-réboxétine par rapport à l'agomélatine seule.

## Revendications

1. Association entre l'agomélatine ou *N*-[2-(7-méthoxy-1-naphtyl)éthyl]acétamide ou un de ses hydrates, formes cristallines et sels d'addition à un acide ou à une base pharmaceutiquement acceptable et un agent inhibiteur de la recapture de la noradrénaline ou permettant d'augmenter la concentration en noradrénaline au niveau extracellulaire.

2. Association selon la revendication 1 dans laquelle l'agent inhibiteur de la recapture de la noradrénaline est la réboxétine.

3. Compositions pharmaceutiques contenant comme principe actif l'agomélatine ou un de ses hydrates, formes cristallines et sels d'addition à un acide ou à une base pharmaceutiquement acceptable en association avec un agent inhibiteur de la recapture de la noradrénaline selon l'une des revendications 1 ou 2 seuls ou en combinaison avec un ou plusieurs excipients pharmaceutiquement acceptables.

4. Compositions pharmaceutiques selon la revendication 3 utiles pour la fabrication d'un médicament pour le traitement du trouble dépressif majeur, des dépressions saisonnières et plus particulièrement des dépressions résistantes, ainsi que dans le traitement de la comorbidité psychiatrique associée au trouble de l'humeur : anxiété, panique, stress, troubles du sommeil.

5. Utilisation d'une association selon l'une des revendications 1 ou 2 pour l'obtention de compositions pharmaceutiques destinées au traitement du trouble dépressif majeur, des dépressions saisonnières et plus particulièrement des dépressions résistantes, ainsi que dans le traitement de la comorbidité psychiatrique associée au trouble de l'humeur : anxiété, panique, stress, troubles du sommeil.

## Claims

1. Association between agomelatine, or *N*-[2-(7-methoxy-1-naphthyl)ethyl]acetamide, or one of its hydrates, crystalline forms and addition salts with a pharmaceutically acceptable acid or base, and a noradrenaline reuptake inhibitor agent or an agent allowing the concentration of noradrenaline at the extracellular level to be increased.

2. Association according to claim 1 wherein the noradrenaline reuptake inhibitor agent is reboxetine.

3. Pharmaceutical compositions comprising as active ingredient, alone or in combination with one or more pharmaceutically acceptable excipients, agomelatine, or one of its hydrates, crystalline forms and addition salts with a pharmaceutically acceptable acid or base, in association with a noradrenaline reuptake inhibitor agent according to claim 1 or 2.

4. Pharmaceutical compositions according to claim 3 for use in the manufacture of a medicament for the treatment of major depressive disorder, seasonal affective disorder and, more especially, resistant depressions, as well as in the treatment of psychiatric co-morbidity associated with mood disorder : anxiety, panic, stress, sleep disorders.

5. Use of an association according to claim 1 or 2 in obtaining pharmaceutical compositions intended for the treatment of major depressive disorder, seasonal affective disorder and, more especially, resistant depressions, as well as in the treatment of psychiatric co-morbidity associated with mood disorder : anxiety, panic, stress, sleep disorders. ,

## Patentansprüche

1. Kombination aus Agomelatin oder *N*-[2-(7-Methoxy-1-naphthyl)-ethyl]-acetamid oder eines seiner Hydrate, Kristallformen und Additionssalze mit einer pharmazeutisch annehmbaren Säure oder Base und einem Mittel zur Inhibierung der Wiederaufnahme von Noradrenalin oder welches die Konzentration an Noradrenalin im extrazellulären Bereich erhöht.

2. Kombination nach Anspruch 1, worin das Mittel zur Inhibierung der Wiederaufnahme von Noradrenalin Reboxetin ist.

3. Pharmazeutische Zubereitungen enthaltend als Wirkstoff Agomelatin oder eines seiner Hydrate, Kristallformen und Additionssalze mit einer pharmazeutisch annehmbaren Säure oder Base in Kombination mit einem Mittel zur Inhibierung der Wiederaufnahme von Noradrenalin nach einem der Ansprüche 1 oder 2, allein oder in Kombination mit einem oder mehreren pharmazeutisch annehmbaren Hilfsstoffen.

4. Pharmazeutische Zubereitungen nach Anspruch 3, die nützlich sind für die Herstellung eines Arzneimittels zur Behandlung von starken depressiven Störungen, saisonal bedingten Depressionen und insbesondere resistenten Depressionen sowie für die Behandlung der psychiatrischen Comorbidität, die mit Gemütsstörungen verknüpft ist: Angst, Panik, Stress, Schlafstörungen.

5. Verwendung einer Kombination nach einem der Ansprüche 1 oder 2 zur Herstellung von pharmazeutischen Zubereitungen, die bestimmt sind für die Behandlung von starken depressiven Störungen, saisonal bedingten Depressionen und insbesondere resistenten Depressionen sowie für die Behandlung der psychiatrischen Comorbidität, die mit Gemütsstörungen verknüpft ist: Angst, Panik, Stress, Schlafstörungen.
